Europäisches Patentamt

European Patent Office

Office européen des brevets

(19)

(11) Veröffentlichungsnummer: **0 262 686**
**A2**

(12) # EUROPÄISCHE PATENTANMELDUNG

(21) Anmeldenummer: 87114425.9

(22) Anmeldetag: 02.10.87

(51) Int. Cl.⁴: **A61L 2/04** , A61L 11/00

(30) Priorität: 02.10.86 DE 3633587

(43) Veröffentlichungstag der Anmeldung:
06.04.88 Patentblatt 88/14

(84) Benannte Vertragsstaaten:
AT BE CH DE ES FR GB GR IT LI LU NL SE

(71) Anmelder: BB-Technik AG
Sonnenstrasse 401
CH-8262 Ramsen(CH)

(72) Erfinder: Die Erfinder haben auf ihre
Nennung verzichtet

(74) Vertreter: Seibert, Rudolf, Dipl.-Ing. et al
Tattenbachstrasse 9
D-8000 München 22(DE)

(54) Vorrichtung zum Desinfizieren oder Verglühen von Injektionsnadeln.

(57) Die Erfindung bezieht sich auf eine Vorrichtung zum Desinfizieren und/oder Verglühen von metallischen, in der Körperheilkunden einsetzbaren Nadeln durch direkten Stromdurchgang.

Im Interesse erhöhter Hygiene wird vorgeschlagen, das zum Vernichten der Nadeln vorgesehene Gerät gleichzeitig zum Aufbewahren von verwendeten, noch auf der Spritze aufgezogenen Nadeln auszubilden, wobei die Einheit als Spritze mit Injektionsnadel dann durch seitliches Verschieben in den Bereich der Elektroden gebracht wird.

EP 0 262 686 A2

## Vorrichtung zum Desinfizieren oder Verglühen von Injektionsnadeln

Die vorliegende Erfindung bezieht sich auf eine Vorrichtung zum Desinfizieren und/oder Verglühen von metallischen, in der Körperheilkunde einsetzbaren Nadeln, vorzugsweise Injektionsnadeln. Es besteht großer Bedarf, Injektionsnadeln, insbesondere in Verbindung von Einmal-Spritzen unbrauchbar zu machen und mit Rücksicht auf Infektionsgefahr auch zu desinfizieren.

Dies einmal, da von Suchtkranken vielfach Wegwerfnadeln usw. gesucht werden, um sie selbst verwenden zu können, und zum anderen, weil sich Reinigungspersonal an derartigen Nadeln leicht verletzen und gegebenenfalls leicht infizieren könnte.

Es sind verschiedene Geräte zum Vernichten derartiger Nadeln bekannt geworden. In neuer Zeit ist ein Gerät bekannt geworden, bei welchem die Nadeln mit Hilfe direkten Stromdurchganges verglüht werden. Dabei sind im Inneren eines mit einer Einstecköffnung versehenen Gehäuses zwei in einem vorgegebenen Abstand angeordnete Elektroden vorgesehen, zwischen die die Nadeln bzw. aufeinanderfolgend kurze Abschnitte der Nadel angelegt und dabei verglüht werden. Dabei wird zwischen die Elektroden eine von einem Niederspannungstransformator abgeleitete relativ niedrige Spannung ausreichenden Stromwertes angelegt, so daß durch den direkten Stromdurchgang durch die Nadelabschnitte diese sehr rasch verglühen. Das verglühte Material tropft dann in einen aus dem Gehäuse herausnehmbaren Behälter. Die verbleibenden Reste sind dabei nicht nur absolut unbrauchbar, sondern durch den Verglühvorgang ist auch jegliche Infektionsgefahr beseitigt.

Die vorliegende Erfindung geht von einem solchen Gerät aus und hat sich zur Aufgabe gestellt, die Gebrauchsfähigkeit und die Einsatzmöglichkeit eines solchen Gerätes zu verbessern und insbesondere auch Verschmutzungs-und damit Infektionsgefahr noch weiter herabzusetzen.

Diese Aufgabe wird mit einer Vorrichtung mit den Merkmalen des Patentanspruches 1 gelöst. Vorteilhafte Ausgestaltungen und Weiterbildungen sind Gegenstand der Unteransprüche.

Bei der Vorrichtung zum Desinfizieren und Verglühen von Injektionsnadeln nach der Erfindung ist neben der eigentlichen Einstecköffnung, wie bei einem bekannten Gerät, in dessen Bereich der Erhitzungs-und Verglühvorgang stattfindet, mindestens ein Schlitz zur weiteren Aufnahme von noch an der Spritze befindlichen Injektionsnadeln vorgesehen, in welchem die einzelnen Spritzen, ohne den Verglühungsvorgang abzuwarten, eingesteckt werden können und von dem sie in den Bereich der Elektroden gebracht werden können, ohne daß die Spritzen selbst wieder aus dem Gehäuse herausgenommen werden müßten.

Dabei ist von der Erkenntnis ausgegangen, daß im praktischen Betrieb, insbesondere, wenn hintereinander mehr Spritzen eingesetzt werden müssen, die Bedienungsperson, also der Arzt oder die Schwester, die Spritzen selbst lediglich ablegen bzw. abstellen wollen, ohne gleich den Verglühablauf auszulösen. Diese Handlungsweise führt dann dazu, daß die einzelnen benutzen Spritzen mit Injektionsnadeln neben das Gerät gelegt werden, bis die Behandlung des Patienten abgeschlossen ist.

Man könnte an sich daran denken, das Gerät dabei so zu entwickeln, daß in einer Vielzahl von Öffnungen die Spritzen unmittelbar abgestellt werden können, wobei sie dann für den Verglühvorgang wieder herausgenommen und in die für das Verglühen vorgesehene Öffnung gesteckt werden müßten.

Dies hätte zwar den Erfolg, daß die Verletzungsgefahr beseitigt wird, da die Spitzen der Nadeln nicht mehr zugänglich sind. Nachteilig an einer derartigen Ausführung wäre aber, daß beim Wiederherausnehmen noch an der Injektionsnadel hängendes Sekret abtropfen könnte, wodurch die Oberfläche des Gerätes, das ja nicht aufgeheizt werden kann, zum Infektionsherd werden könnte.

Bei der Vorrichtung nach der Erfindung wird die zu vernichtende, noch auf dem Spritzenkörper aufgesetzte Nadel einfach seitlich in den Bereich der Elektroden verschoben, wo sie dann unmittelbar verglüht. Dabei ist im Bereich der Mittenöffnung die Einführöffnung so groß gehalten, daß auch der Kopf der Nadel nach unten geht, während zunächst im Schlitz die Nadel mit dem Kopf gehalten wird, damit sie sicher nicht in den Behälter unverglüht und deshalb infiziert gelangen kann.

Um beim seitlichen Verschieben der Nadeln die Nadel sicher als Ganzes verglühen zu können, sind gemäß einer vorteilhaften Weiterbildung des Erfindungsgedankens die im Inneren des Gehäuses vorzusehenden Elektroden dachförmig mit im Bereich der eigentlichen Verglühöffnung höchsten Spitze angeordnet, wobei die Steigung zu gewählt ist, daß die in der Praxis vorkommende längste Nadel sicher auf die untere der beiden Elektroden aufgleitet, wodurch dann durch weiteres Verschieben sukzessiv die Nadel abbrennt.

Bei einer anderen Ausführungsmöglichkeit wird erfindungsgemäß vorgeschlagen, die Einheit der beiden Elektroden federn im Gehäuse so aufzuhängen, daß die Einheit von unten nach oben gegen die Verglüheinführöffnung gedrückt wird wobei eine Kombination mit schräger Auflauffläche für die Nadelspitze, die von der Seite kommend auf die Elektrode aufgeschoben wird, vorgesehen werden kann.

Um einen Austritt auch nur geringster Mengen eines absolut unschädlichen Metalldampfes aus dem Gehäuse selbst sicher zu verhindern, wird gemäß einer anderen Weiterbildung des Erfindungsgedankens vorgeschlagen, die Öffnung im Bereich der Verglühelektroden aber auch der Führungsschlitze mit einer Borstenabdeckung zu versehen, durch die jede Nadel sehr einfach geschoben und in der sie auch seitlich verschoben werden kann. Dabei ist es vorteilhaft, die Borsten aus Metall zu machen, da in diesem Fall sowohl eine Kontaktgabe für den Verglühvorgang als auch ein Desinfizieren durch unmittelbaren Stromdurchgang, in dem die Versorgungsquelle vorübergehend an die Metallborsten zum unmittelbaren Stromdurchgang angeschlossen werden, ermöglicht wird.

Die vorstehenden Angaben werden zum besseren Verständnis im folgenden in Verbindung mit den anliegenden Zeichnungen ergänzt und vertieft.

In den Zeichnung zeigen:

Fig. 1 Gestaltung und Formgebung eines Gehäuses für eine Vorrichtung nach der Erfindung,

Fig. 2 eine Teilschnittdarstellung längs der Linie II-II in Fig. 1,

Fig. 3 schematisch die Zuordnung der Elektroden längs eines Schnittes III-III in Fig. 2, und

Fig. 4 den prinzipiellen Aufbau einer Elektrodenanordnung mit im Gehäuse federnd abgestützter Elektrodeneinheit.

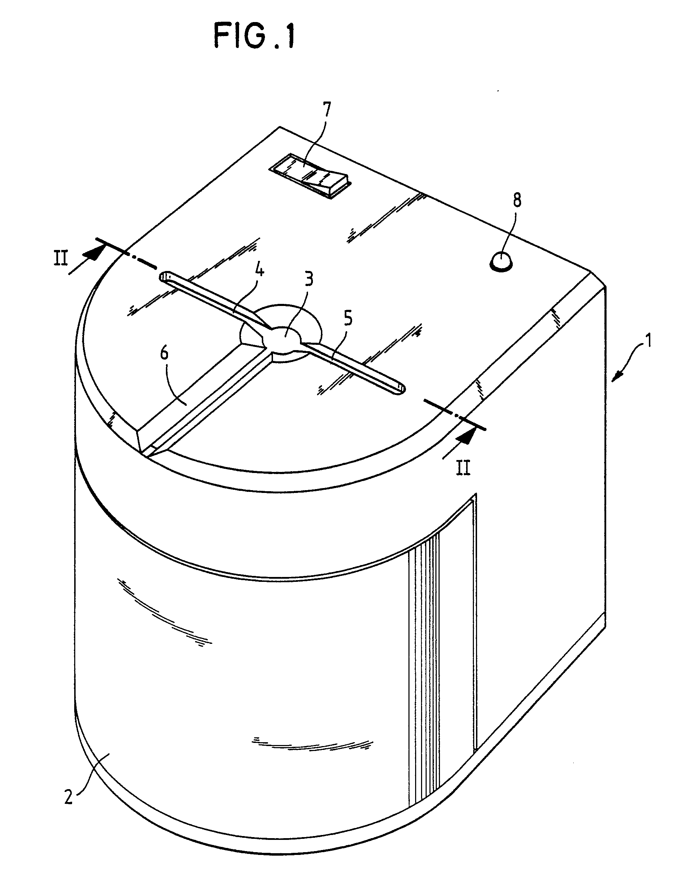

Die Fig. 1 zeigt in einer perspektivischen Ansicht Form und Oberflächengestaltung einer Vorrichtung zum Verglühen von Injektionsnadeln.

Die gesamte Vorrichtung besteht bis auf die im einzelnen noch zu beschreibenden Zugangsöffnungen aus einem geschlossenen Gehäuse 1. In das Gehäuse ist ein Aufnahmebehälter 2 von vorne eingeschoben, in welchem die Rückstände der zu verglühenden Nadeln aufgefangen werden. Der Aufnahmebehälter 2 ist herausnehmbar, um ihn entleeren und reinigen zu können. Hierzu kann ein Halteknopf oder drgl. vorgesehen werden. Bei dem dargestellten Ausführungsbeispiel ist eine Riffelung angedeutet, die ein sicheres Halten des Aufnahmebehälter zwischen Daumen und Zeigefinger garantiert.

Auf der Oberseite ist eine Einführöffnung 3 vorgesehen, durch die eine auf einem Spritzenbehälter noch aufgezogenen Injektionsnadel zum Verglühen in das Gehäuse, und dort, wie noch gezeigt wird, in den Bereich von Elektroden geführt werden kann. Dabei ist die lichte Öffnung der Einführöffnung 3 so groß gewählt, daß auch der Kopf einer Injektionsnadel selbst mit einer gegebenenfalls vorhandenen Umbördelung hindurchgesteckt werden kann.

Auf beiden Seiten der Öffnung 3 sind Schlitze 4 und 5 vorgesehen, die in ihrer Breite so gehalten sind, daß eine Injektionsnadel leicht eingeführt werden kann, daß aber die Nadel mit ihrem Kopf am Rand des Schlitzes hängen bleibt.

Die Schlitze dienen dazu Spritzen mit Injektionsnadeln abstellen zu können, ohne einen Verglühvorgang abwarten zu müssen.

Um das Verständnis zu erleichtern, sind die Maßstäbe etwas verzerrt dargestellt, d. h. bei einer praktischen Ausführungsform beträgt die Länge der Schlitze 4 und 5 ein Vielfaches des Durchmessers der Einstecköffnung 3.

Durch die Wahl der lichten Weiten wird garantiert, daß bei einem seitlichen Abstellen einer Spritze mit Injektionsnadel die Nadel sicher nicht vom Spritzenkörper in den Aufnahmebehälter 2, der sich unterhalb des Schlitzes befindet, fallen kann, sondern so lange mit dem oberen Rand auf der Oberseite des Gehäuses bleibt, bis die Nadel in den Bereich der Aufnahmeöffnung, wo sich im Inneren die Elektroden befinden, seitlich geschoben wurde.

Von der Einführöffnung 3 nach vorne ist ein Führungsschlitz 5 vorgesehen, der so ausgebildet ist, daß seine Ränder sich zwischen Kopf einer Injektionsnadel und Spritzenkörper einschieben, so daß der Rest der an sich verglühten Nadel in dem Behälter abgezogen wird und der Spritzenkörper als solcher weggenommen werden kann.

Die Oberseite des Gehäuses 1 trägt außerdem einen nur schematisch angedeuteten Ein-und Ausschalter 7 sowie eine Kontrollleuchte 8.

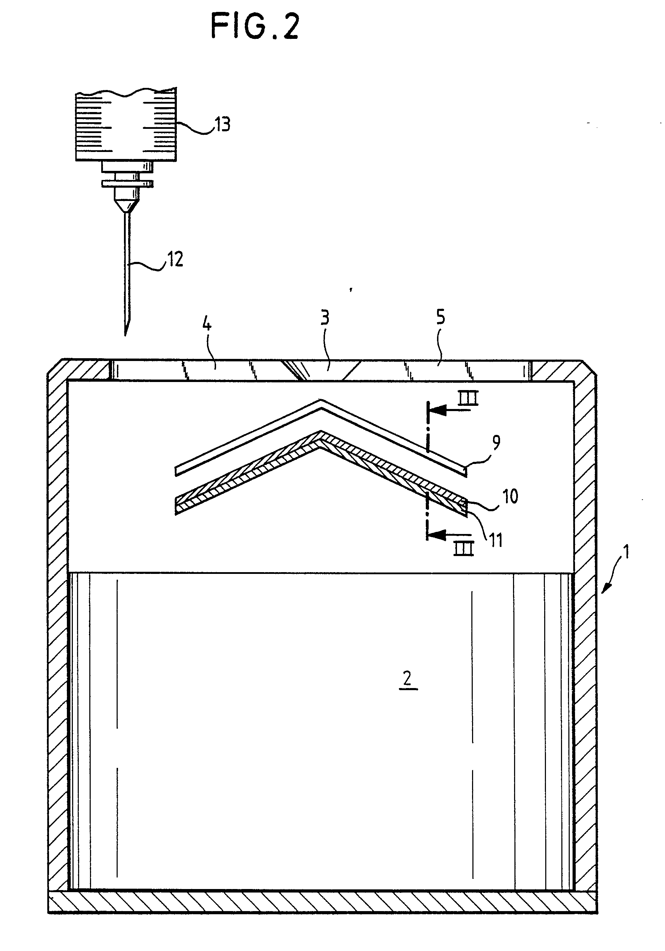

In Fig. 2 ist ein Schnitt durch das Gehäuse längs der Linie II-II in Fig. 1 wiedergegeben wobei im Inneren nur die Teile dargestellt sind, die für das Verständnis der Erfindung von Bedeutung sind.

Das Gehäuse 1 nimmt im unteren Teil den Aufnahmbehälter 2 auf. Auf der Oberseite ist die Einführöffnung 3 vorgesehen, an die sich die beiden Längsschlitze 4 und 5 entsprechend anschließen.

Im Inneren des Gehäuses sind dann zwei Elektroden, nämlich eine obere Elektrode 9 und eine untere Elektrode, die aus zwei Schichten 10 und 11 besteht, vorgesehen.

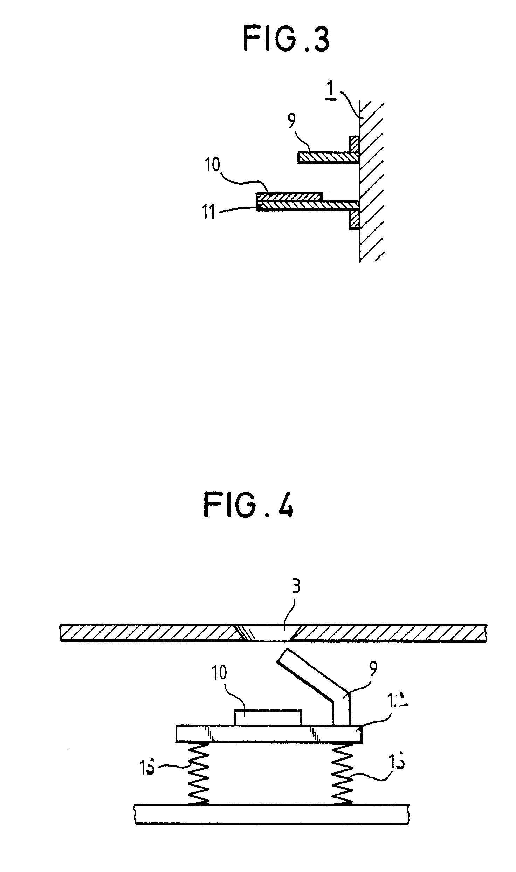

Die Erstreckung der Elektroden in den Raum in Fig. 3 anschaulich gezeigt. Dabei ist die Elektrode 9, also die obere Elektrode an der Rückwand befestigt und erstreckt sich in den Bereich der Öffnung 3 bzw. der seitlichen Schlitze 4 und 5 bis ziemlich genau in deren Mitte, so daß eine Nadel an der Vorderkante der Elektrode 9 vorbeigleiten kann. Die andere Elektrode trägt auf ihrer Oberseite einen gut leitenden Belag, beispielsweise auch aus Graphit, auf den sich dann die Spitze der Nadel abstützt, wodurch dann ein Kurzschluß zwischen der Elektrode 10 und der Elektrode 9 über den entsprechenden Abschnitt der Injektionsnadel zustande kommt, so daß diese stark erhitzt und verglüht wird. Entsprechend dem Fortgang des Glühvorganges wird dann die Nadel entsprechend nachgeschoben, bis sie in den Bereich des Injektionsnadelkopfes verglüht ist.

Die Elektroden selbst sind nach Art eines Daches an der Rückwand befestigt, wobei die höchste Erstreckung im Bereich der Einführöffnung 3 liegt, während die seitlichen Enden soweit heruntergezogen sind, daß bei einem seitlichen Verschieben einer Injektionsnadel 12, die beispielsweise in den Schlitz 4 eingeführt wurde, diese mit ihrer unten liegenden Spitze auf die Elektrode 10 aufgleitet und dann längs der Elektrode 9 und auf der Elektrode 10 beim seitlichen Verschieben der Spritze 13 in den Bereich der Einführöffnung gelangt. Erst im Bereich der Einführöffnung 3 kann dann die Einheit aus Spritzenkörper 13 und Injektionsnadel 12 noch tiefer in das Gehäuse eingeführt werden, wodurch ein endgültiges Abbrennen der Injektionsnadel bis zum Injektionsnadelkopf erfolgt.

Die Ausgestaltung der Elektroden in Dachform ist nur beispielhaft. Es kann jede Art einer Elektrodenführung an der Rückwand befestigt werden, wobei dafür zu sorgen ist, daß eine seitlich verschobene Nadel sicher auf die untere Elektrode aufgleitet und nicht etwa einen Kurzschluß im oberen Bereich bildet, wodurch die Spitze in den Auffangbehälter fallen würde. Dabei können sowohl halbrunde Formen oder aber auch eine federnde Aufhängung der aus oberen und unteren Elektrode bestehenden Einheit vorgesehen werden, wobei die Aufhängung die Elektrodeneinheit federnd nach oben drückt oder aber auch ziehen soll, und zwar in einem solchem Maße, daß die obere Elektrode 9 möglichst nahe zur oberen Wand des Gehäuses kommt, um eine eingeführte Injektionsnadel möglichst vollständig verglühen zu können.

Eine entsprechende Ausführung ist rein schematisch in Fig. 4 gezeigt, wo die Elektrodeneinheit bestehend aus oberer Elektrode 9 und unterer Elektrode 10 auf einem Support 12 befestigt ist. Das Support 12 ist mit Federn 13 abgestützt und wird mit den darauf befindlichen Elektroden nach oben gegen die Öffnung 3 gedrückt.

Beim Einführen einer Nadel von oben wird die Bedienungsperson die Einheit nach unten drücken und diese drückt dann mit dem Fortgang des Schmelzvorganges nach oben, bis sie sich wieder im Bereich der Einsteköffnung 3 befindet.

Die Erfindung wurde anhand einiger schematisch erläuterter Konstruktionseinzelheiten beschrieben. Für den auf dem Sachgebiet tätigen Fachmann ergeben sich dabei eine Reihe von Ausgestaltungen und Weiterbildungen, die im Rahmen der Erfindung realisiert werden können.

So ist es beispielsweise ohne weiteres möglich, die seitliche Verschiebung der mit Injektionsnadeln noch versehenen Spritzenkörper nicht längs einer Geraden, sondern eines Kreisbogens oder dgl. vorzunehmen, wobei auch Unebenheiten, Klemmkörperstützen usw. für die Spritzenkörper selbst vorgesehen werden können, um mit Sicherheit ein Umkippen auch längerer Spritzen zu vermeiden. Auch eine Kombination mit mechanisch wirkenden Nadelzerstörern im Inneren des Gehäuses, die beispielsweise beim Erhitzungsvorgang, oder nach dem Erhitzungsvorgang, auch wenn nur ein Desinfektionserhitzen eingestellt wird, die Nadeln verbiegen und damit zusätzlich unbrauchbar machen.

Mit einer Vorrichtung nach der Erfindung kann beispielsweise auch im Klinikbetrieb eine Schwester eine Mehrzahl von Behandlungen hintereinander durchführen und dabei die einzelnen Spritzen nebeneinander in dem Gerät selbst abstellen. Dabei sei darauf hingewiesen, daß der Maßstab in Fig. 2 bewußt im Intersse des Verständnisses insoweit verzerrt ist, als die Elektroden so dargestellt sind, wie wenn sie sich über die ganze Länge der Einsteckschlitze 4 und 5 erstrecken würde. In der Praxis ist dies keineswegs notwendig und sinnvoll, sondern es genügt, daß der Glühvorgang bei einem seitlichen Führen der Spritze kurz vor Erreichen der Einführöffnung 3 eingeleitet und dort abgeschlossen wird.

Da aber eine Mehrzahl von Spritzen abgestellt werden kann, ist es gemäß einer besonderen Ausgestaltung der Erfindung vorteilhaft, eine Überwachungsschaltung vorzusehen, die bei einem zu rasch aufeinanderfolgenden Verglühen von mehreren Injektionsnadeln eine Überlastung der Stromversorgung verhindert, beispielsweise dadurch, daß zwischen den einzelnen Glühvorgängen eine Pause über eine Zeitsteuerung erzwungen wird. Auch eine Überwachung des Glühstromes selbst dahingehend, daß die Vorrichtung dann abgeschaltet wird, wenn mehr als eine Nadel in den Bereich der Elektroden gelangt, kann über diese Überwachungsschaltung realisiert werden.

Soll, was im Rahmen der Erfindung mit einer derartigen Vorrichtung durchaus realisiert werden kann, eine Injektionsnadel nur ausgeglüht und nicht etwa abgeschmolzen werden, dann kann dies mit einer Stromüberwachung durchgeführt werden, die den an die Nadel angelegten Strom auf einen vorgegebenen Wert begrenzt.

Derartige Schaltungen sind hinreichend bekannt und vom Fachmann unmittelbar und ohne erfinderisches Dazutun realisierbar.

Dabei muß in die Überwachung der Elektrodenabstand, der im Fall des reinen Ausglühens größer sein kann als bei einem Schmelzen, mit berücksichtigt werden.

Auch - einfache - Temperatursensoren, die beispielsweise auf das beim Glühen ausgesandte Rotlicht ansprechen und den Strom anschließend abschalten, können im Rahmen der Erfindung unmittelbar eingesetzt werden.

## Ansprüche

1. Vorrichtung zum Desinfizieren und/oder Verglühen von metallischen, in der Körperheilkunde einsetzbaren Nadeln, vorzugsweise Injektionsnadeln durch direkten Stromdurchgang (Kurzschlußstrom), der die Nadeln zumindest auf Glühtemperatur, vorzugsweise auf Schmelztemperatur erhitzt, bestehend aus einem mit mindestens einer Öffnung zum Einstecken der Nadel versehenen Gehäuse, in welchem in Zuordnung zur Öffnung eine erste Elektrode, an welche ein Nadel zum Erhitzen unter Berührung vorbeiführbar ist, und eine zweite Elektrode, auf der die zu erhitzende Nadel auftrifft, vorgesehen sind, wobei zwischen beiden Elektroden eine Gleich-oder Wechselspannung angelegt ist, und mit einem aus dem Gehäuse herausnehmbaren Aufnahmebehälter zur Aufnahme von Schmelzresten und/oder ausgeglühter, gegebenenfalls verformter Nadeln oder Nadelreste,
dadurch gekennzeichnet,
daß auf der Oberseite des Gehäuses (1) eine Mehrzahl von Öffnungen (4, 5) zur gleichzeitigen Ablage von mehreren mit zu vernichtenden Nadeln, versehenen Spritzenkörper, von denen einige von ihnen neben dem Bereich der im Gehäuse vorgesehenen Elektroden liegen.

2. Vorrichtung nach Anspruch 1,
dadurch gekennzeichnet, daß in der Oberseite des Gehäuses mindestens ein zur Aufnahme mehrerer noch am Spritzenkörper befestigter Nadeln geeigneter Längsschlitz (4,5) mit einer Breite vorgesehen ist, daß die Nadeln bis zum Nadelkopf einführbar sind, daß an einem Ende des Längsschlitzes eine Einführöffnung (3) zur Aufnahme der Nadeln einschließlich Nadelkopf vorgesehen ist, und daß im Bereich der Einführöffnung zumindest ein Abschnitt der Elektroden (9, 10) vorgesehen ist.

3. Vorrichtung nach Anspruch 1 oder 2,
dadurch gekennzeichnet, daß die erweiterte Einführöffnung (3) für Nadel einschl. Kopf in einem mittleren Bereich des Gehäuses angeordnet ist und daß beidseitig von dieser Öffnung (3) Schlitze (4, 5) zur Aufnahme der Nadeln bis zum Nadelkopf vorgesehen sind.

4. Vorrichtung nach Anspruch 3,
dadurch gekennzeichnet, daß im Inneren des Gehäuses (1) die Elektroden dachförmig ausgebildet sind, wobei die größte Annäherung der Elektroden an die Oberfläche im Bereich der erweiterten Einführöffnung vorgesehen ist.

5. Vorrichtung nach Anspruch 1 bis 4,
dadurch gekennzeichnet, daß die Breitenerstreckung der Elektroden so gewählt ist, daß mindestens eine Nadel mit Spritzenkörper am Ende des Einführschlitzes einsetzbar ist, ohne mit einer Elektrode in Berührung zu kommen.

6. Vorrichtung nach einem der Ansprüche 1 bis 4,
dadurch gekennzeichnet, daß der erweiterten Einführöffnung (3) ein an sich bekannter Abstreifschlitz (6) zugeordnet ist.

7. Vorrichtung nach einem der Ansprüche 1 bis 6,
dadurch gekennzeichnet, daß zumindest im Bereich der erweiterten Einführöffnung eine Ringelektrode zur Kontaktgabe mit dem Nadelkopf vorgesehen ist.

8. Vorrichtung nach Anspruch 7,
dadurch gekennzeichnet, daß die Öffnungen im Inneren eine Bürstenabdeckung aufweisen, durch den die Nadeln einführbar sind.

9. Vorrichtung nach Anspruch 8,
gekennzeichnet durch die Verwendung von Metallborsten für die Bürstenabdeckung.

10. Vorrichtung nach einem der Ansprüche 1 bis 9,
dadurch gekennzeichnet, daß die Elektroden im Inneren des Gehäuses als Einheit federnd mit einem Federdruck oder -zug nach oben gegen die erweiterte Öffnung aufgehängt und/oder abgestützt sind.

11. Vorrichtung nach einem der Ansprüche 1 bis 10,
dadurch gekennzeichnet, daß ein Zeit-oder Kontrollglied vorgesehen ist.

12. Vorrichtung nach Anspruch 11, dadurch gekennzeichnet, daß abhängig vom Zeitglied die Aufeinanderfolge von Erhitzungs-und/oder Verglühtakten unter Einhaltung vorgegebener Mindestpausen steuerbar ist.

13. Vorrichtung nach Anspruch 11, dadurch gekennzeichnet, daß abhängig vom Steuerglied die Stromversorgung immer dann abschaltbar ist, wenn die Elektroden von mehr als einer Nadel überbrückt sind.

14. Vorrichtung nach einem der vorhergehenden Ansprüche, gekennzeichnet durch die Kombination mit einer an sich bekannten mechanisch arbeitenden Verformeinrichtung für ausgeglühte Nadeln innerhalb des Gehäuses.

# FIG.1

# FIG.2

# FIG.3

# FIG.4